# FASCICULE DE BREVET EUROPEEN

(11) **EP 0 542 596 B1**
(45) Date de publication et mention de la délivrance du brevet: **28.08.1996**
(21) Numéro de dépôt: 92402950.7
(22) Date de dépôt: 30.10.1992
(51) Int. Cl.: C07C 41/38, C07C 43/04

(54) **Procédé de séparation d'éthyl tertiobutyl éther et d'éthanol**
Verfahren zur Trennung von Ethyl-tert-butyl-ether und Ethanol
Method of separating ethyl-tert-buthyl-ether and ethanol

(30) Priorité: 12.11.1991 FR 9114062
(43) Date de publication de la demande: 19.05.1993
(73) Titulaire: INSTITUT FRANCAIS DU PETROLE, F-92502 Rueil-Malmaison (FR)
(72) Inventeur: Pucci, Annick, F-78290 Croissy Sur Seine (FR); Mikitenko, Paul, F-78590 Noisy Le Roi (FR); Zuliani, Massimo, F-92502 Rueil Malmaison (FR)

(56) Documents cités:
- EP-A- 0 071 032
- US-A- 5 015 783
- CHEMICAL ABSTRACTS, vol. 44, no. 4, 25 Février 1950, Columbus, Ohio, US; abstract no. 1526c, page 1950 ;colonne 2 ;
- KIRK-OTHMER 'Encyclopedia of Chemical Technology 3rd edition Supplement volume' 1984 , JOHN WILEY & SONS; NEW YORK; EUA

## Description

L'invention concerne la fabrication de l'éthyl tertiobutyl éther (en abrégé ETBE) et plus particulièrement sa séparation à partir de mélanges avec l'éthanol.

On sait que l'éthyl tertiobutyl éther, comme le méthyl tertiobutyl éther (en abrégé MTBE), peut être utilisé comme additif à haut indice d'octane pour les essences sans plomb ou à teneur en plomb réduite. On peut prévoir d'ajouter l'ETBE aux essences à des concentrations allant par exemple jusqu'à environ 15 % en volume.

Un procédé de fabrication du MTBE consiste à réaliser une réaction d'addition de méthanol sur de l'isobutène, par exemple contenu dans une coupe C₄ de vapocraquage, de craquage catalytique ou de déshydrogénation d'isobutane. Après réaction, le méthanol résiduel est en général séparé par distillation azéotropique avec les hydrocarbures en C₄, ce qui permet d'obtenir assez aisément le MTBE avec un degré de pureté convenable pour être additionné aux essences.

La fabrication de l'ETBE peut être réalisée par un procédé analogue, dans lequel l'éthanol remplace le méthanol. Un tel procédé est décrit par exemple dans "L'ETBE, un avenir pour l'éthanol" de A. FORESTIERE, B. TORCK et G. PLUCHE, communication faite à la Conférence sur la Biomasse pour l'Energie et l'Industrie, Lisbonne, 9-13 Octobre 1989 et dans "MTBE/ETBE, an Incentive Flexibility for Refiners", par A. FORESTIERE et coll., communication faite à la "Conférence on Oxygenated Fuels in Europe", Londres, 22-23 Mai 1990.

Cependant, par un tel procédé, contrairement au cas du MTBE, après élimination des hydrocarbures en C₄, la quasi-totalité de l'éthanol résiduel se retrouve en mélange avec l'ETBE produit. L'existence d'un azéotrope éthanol-ETBE à 21 % en poids d'éthanol à la pression atmosphérique, bouillant à 66,6°C, rend difficile la séparation de l'ETBE avec un degré de pureté suffisant pour satisfaire les spécifications concernant la teneur en éthanol des essences. Ainsi, la teneur en éthanol de l'ETBE doit être généralement comprise entre 0,5 et 10 % en poids. Avantageusement, l'ETBE devra être purifié de 2 % en poids d'éthanol pour être acheminé à la raffinerie.

Ainsi, pour que l'ETBE puisse concurrencer le MTBE comme additif améliorant l'indice d'octane des essences sans plomb, il était particulièrement souhaité de trouver un procédé de séparation économiquement attractif. C'est ce que l'invention propose.

L'invention a donc pour objet un procédé de séparation de l'ETBE à partir de mélanges qu'il forme avec l'éthanol, et plus particulièrement à partir des mélanges ETBE-éthanol issus de la réaction de l'éthanol sur une coupe C₄ de vapocraquage catalytique ou de déshydrogénation d'isobutane.

L'invention a également pour objet un procédé de fabrication de l'ETBE incluant une telle opération de séparation et dans lequel l'éthanol est recyclé au réacteur d'éthérification.

Le procédé de séparation de l'ETBE et d'éthanol de l'invention s'adresse en général aux mélanges constitués essentiellement d'éthanol et d'ETBE en proportions variées, et plus particulièrement aux mélanges issus de la réaction d'addition d'éthanol sur l'isobutène contenu dans une coupe C₄ de vapocraquage, de craquage catalytique ou de déshydrogénation d'isobutane, qui contiennent en général de 5 à 50 %, le plus souvent de 10 à 30 %, en poids d'éthanol.

Le procédé de séparation d'éthyl tertiobutyl éther de l'invention basé globalement sur la mise en oeuvre d'une extraction à l'eau, d'une concentration et d'une distillation hétéroazéotropique dans deux colonnes couplées avec un décanteur en tête, peut être défini d'une manière générale par le fait qu'il comprend:
(1) une étape d'extraction dans laquelle on introduit une charge consistant essentiellement en un mélange d'éthyl tertiobutyl éther et d'éthanol dans une zone d'extraction dans laquelle on introduit comme solvant d'extraction de l'eau et à partir de laquelle on recueille un raffinat consistant essentiellement en éthyl tertiobutyl éther saturé en eau et un extrait consistant en un mélange éthanol-eau contenant une faible proportion d'éthyl tertiobutyl éther;
(2) une étape de concentration dans laquelle l'extrait issu de l'étape (1) est envoyé dans une zone de concentration à partir de laquelle on recueille en tête un distillat proche de la composition azéotropique éthanol-eau, en fond un résidu consistant essentiellement en eau, et on soutire une phase consistant essentiellement en éthyl tertiobutyl éther, qui est réunie avec le raffinat de l'étape d'extraction (1); et
(3) une étape de distillation hétéroazéotropique utilisant l'éthyl tertiobutyl éther comme agent azéotropant, dans laquelle on envoie le distillat de ladite étape de concentration dans une première zone de distillation à partir de laquelle on recueille un résidu consistant essentiellement en éthanol, le flux sortant en tête étant réuni au flux de tête d'une seconde zone de distillation, le flux résultant étant condensé et envoyé dans un décanteur où se séparent une phase supérieure riche en éthyl tertiobutyl éther envoyée en reflux en tête de ladite première zone de distillation et une phase inférieure riche en eau renvoyée en reflux en tête de ladite seconde zone de distillation, dont le résidu consitant essentiellement en eau, qui est envoyée, après réunion avec le résidu de ladite zone de concentration et éventuel appoint, en alimentation en tête de ladite zone d'extraction, de l'éthyl tertiobutyl éther étant introduit dans le décanteur lors de la mise en service du procédé.

On peut noter que le document KIRK-OTHMER "Encyclopedia of Chemical Technology 3rd Edition Supplement volume" pp 150-153 décrit la séparation de mélanges eau-éthanol par distillation azéotropique, les agents azéotropants cités étant le benzène, le pentane, le cyclohexane et le diéthyléther. L'emploi de l'ETBE n'est ni décrit ni suggéré par ce document.

Le document US-A-5 015 783 décrit un procédé de production d'éthers par réaction d'alcools d'oléfines dans lequel l'effluent de la zone d'éthérification qui comprend l'éther, l'acool, les hydrocarbures non-réactifs et non réagis et de l'eau passe dans une colonne de distillation, dans laquelle sont séparés en fond l'éther et en tête, l'alcool, les hydrocarbures et l'eau; la condensation de l'effluent de tête donne naissance à une phase liquide hydrocarbonée, qui est en partie renvoyée en reflux de la colonne de distillation; et à une phase aqueuse (alcool + eau) qui est en partie recyclée à l'entrée de la zone d'éthérification et en partie combinée à l'effluent de tête de la colonne de distillation avant son refroidissement.
L'extraction à l'eau de l'alcool des mélanges ETBE-éthanol n'est ni décrite ni suggérée par ce document.

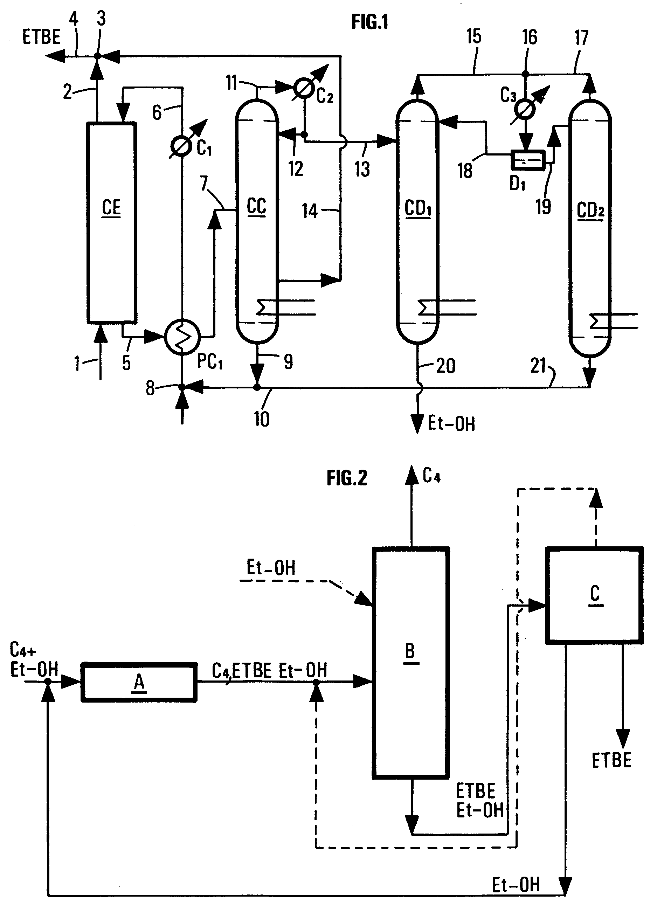

Les conditions de réalisation du procédé de l'invention sont décrites de façon plus détaillée ci-après, en liaison avec la figure 1 annexée. Les pressions indiquées dans la présente description sont des pressions absolues, exprimées en bars (1 bar = 0,1 MPa).

La charge consitant en le mélange d'ETBE et d'éthanol à séparer est envoyée par la ligne 1 dans une colonne d'extraction CE sous une pression p₁ de 1 à 2 bars, en général à une température de 50 à 70°C, par exemple d'environ 60°C, au fond de ladite colonne. L'eau qui constitue le solvant d'extraction est amenée par la ligne 6 en tête de colonne dans les mêmes conditions de température et de pression. On utilise en général un rapport d'extraction de 2 à 5 moles d'eau par mole de charge.

La phase "raffinat" constituée d'ETBE saturé en eau sort en tête par la ligne 2 et est mélangée en 3 avec la phase ETBE, elle est aussi saturée en eau, qui a décanté sur les plateaux de la colonne CC de concentration du mélange éthanol-eau, d'où elle est soutirée par la ligne 14. Le mélange des deux courants est recueilli par la ligne 4.

L'ETBE ainsi séparé a en général une pureté d'au moins 98,8 % en poids. Il contient des traces d'eau. Si l'on souhaite obtenir l'ETBE pur, il y a lieu de prévoir une déshydratation subséquente, par tout moyen approprié, par exemple un séchage sur tamis.

Soutirée au fond de la colonne d'extraction CE par la ligne 5, la phase "extrait", qui contient environ 75 % en poids d'eau, environ 22 % en poids d'éthanol et environ 3 % en poids d'ETBE est chauffée, de préférence par passage dans l'échangeur de préchauffage PC 1, et est envoyée par la ligne 7 dans la colonne de concentration CC, à la pression p₁. La colonne de concentration CC opère en général entre une température de fond de 100 à 120°C et une température de tête de 78 à 85°C. La distillat qui sort par la ligne 11 consiste en un mélange dont la composition est proche de celle de l'azéotrope éthanol-eau à la pression p₁ (soit environ 9,1 % en poids d'eau et environ 90,9 % en poids d'éthanol), mais qui contient encore une très faible proportion d'ETBE. Ce distillat est condensé dans le condenseur C₂, le condensat étant en partie renvoyé, à titre de reflux, en tête de ladite colonne de concentration par la ligne 12 et en partie envoyé, par la ligne 13, vers la colonne CD1 de distillation hétéroazéotropique sous la pression p₁.

On soutire par la ligne 14 une phase ETBE, saturée en eau, qui a décanté sur les plateaux de la colonne de concentration CC. Comme déjà décrit plus haut, cette phase ETBE est mélangée en 3 avec la phase ETBE sortant en tête de la colonne d'extraction CE par la ligne 2.

En fond de la colonne de concentration CC, on soutire par la ligne 9 un effluent constitué essentiellement d'eau et de traces d'éthanol ; il est mélangé en 10 avec le résidu de la colonne de distillation CD 2, amené par la ligne 21. Le courant résultant reçoit éventuellement un appoint d'eau en 8. Il est refroidi par exemple par passage dans l'échangeur PC 1, et dans le condenseur C1, avant d'alimenter la colonne d'extraction CE par la ligne 6, comme déjà indiqué plus haut.

Le mélange éthanol-eau sortant en tête de la colonne de concentration CC contenant encore une très faible proportion d'ETBE est ensuite séparé en éthanol et eau dans une zone de distillation hétéroazéotropique utilisant l'ETBE comme agent azéotropant.

Le mélange éthanol-eau à séparer alimente par la ligne 13 une première colonne de distillation CD 1 opérant en général sous la pression p₁, entre une température de fond de 78 à 85°C et une température de tête de 64 à 70°C.

Le distillat sortant en tête par la ligne 15 a une composition voisine de celle de l'azéotrope ternaire éthanol-eau - ETBE à la pression p₁, soit, sous 1 bar, environ 13 % en moles d'eau, 27 % en moles d'éthanol et 60 % en moles d'ETBE, est réuni en 16 avec le distillat sortant en tête de la deuxième colonne de distillation CD 2, par la ligne 17 avec une composition d'environ 82 % en moles d'éthanol, 17 % en moles d'eau et 1 % en moles d'ETBE. Le flux résultant est ensuite envoyé dans le condenseur C₃ où il est refroidi en général à une température d'environ 45 à 65°C, plus particulièrement à une température d'environ 54°C, puis amené dans le décanteur D1 où il se sépare en deux phases : une phase riche en ETBE qui est renvoyée par la ligne 18, à titre de reflux, en tête de la colonne CD1 ; et une phase riche en eau qui, par la ligne 19, alimente la colonne CD2.

La colonne CD2 opère en général à la pression p₁, entre une température de fond de 100 à 120°C et une température de tête de 94 à 110°C.

Au fond de la colonne CD1, par la ligne 20, on recueille l'éthanol avec une pureté de 99 % en moles, le complément consistant en eau et ETBE. La perte d'ETBE qui en résulte peut être compensée par un appoint effectué par exemple au niveau du condenseur C₃.

Au fond de la colonne CD2, par la ligne 21, sort de l'eau pratiquement pure (par exemple 99,9 % en poids). Elle est réunie en 10 à l'effluent de fond de la colonne de concentration CC pour aller alimenter en eau la colonne d'extraction CE, par la ligne 6.

L'ensemble formé par les deux colonnes de distillation CD1 et CD2 et le décanteur D1, agencés comme décrit plus haut, peut, considéré séparément, constituer un procédé de séparation d'eau et d'éthanol à partir de leurs mélanges, en particulier de composition proche de l'azéotrope à la pression considérée.

Le procédé de séparation de l'ETBE de l'invention, tel qu'il a été décrit ci-dessus, peut être avantageusement intégré dans un procédé complet de fabrication d'ETBE par éthérification au moyen d'éthanol de l'isobutène contenu dans une coupe C₄ de vapocraquage, de craquage catalytique ou de déshydrogénation d'isobutane.

Le procédé de fabrication d'ETBE, décrit en liaison avec le schéma de la Figure 2 annexée, comprend alors les étapes suivantes : dans une zone A, on met en contact dans des conditions de réaction de l'éthanol et une coupe C₄ de vapocraquage, de craquage catalytique ou de déshydrogénation d'isobutane ; le produit issu de la zone réactionnelle A contient principalement de l'ETBE, de l'éthanol, des hydrocarbures en C₄ autres que l'isobutène et de l'isobutène non réagi. Ce produit est envoyé dans une zone de distillation B, où sont séparés en tête les hydrocarbures en C₄ très appauvris en isobutène et, en fond, un mélange d'ETBE et d'éthanol. Si l'on souhaite une élimination plus complète de l'isobutène, on peut mettre en oeuvre, au lieu d'une simple distillation B, une distillation réactive B', mettant en jeu un appoint d'éthanol, comme indiqué en pointillés sur la Figure 2. Dans tous les cas, le mélange d'ETBE et d'éthanol, recueilli en fond de zone B est envoyé dans une zone C, où est réalisé le procédé de séparation de l'invention.

A partir de la zone C, l'éthanol purifié recueilli est avantageusement recyclé comme appoint vers la zone de réaction A et/ou vers la zone de distillation réactive B'.

### EXEMPLE

L'exemple suivant illustre l'invention

La charge à traiter contient 80 % en poids d'ETBE et 20 % en poids d'éthanol.

On utilise :
- un extracteur liquide-liquide (colonne CE) en acier inoxydable de 45 mm de diamètre et ayant 10 plateaux perforés espacés de 10 cm ;
- une première colonne à distiller (colonne de concentration) en acier inoxydable de 100 mm de diamètre et comportant 16 plateaux perforés espacés de 5 cm ;
- une colonne à distiller CD1 en acier inoxydable de 50 mm de diamètre et comportant 48 plateaux perforés à déversoir espacés de 5 cm ;
- une colonne à distiller CD2 en acier inoxydable de 25 mm de diamètre et comportant 6 plateaux perforés à déversoir espacés de 5 cm ; et un décanteur de 15,5 litres.
Les appareils sont agencés comme l'indique le schéma de la figure 1 ci-annexée.

La charge est introduite dans l'extracteur, au niveau du 10ème plateau (les plateaux étant comptés de haut en bas), sous un débit de 8,21 kg/heure, à une pression de 1 bar. L'eau est introduite au niveau du premier plateau, à la température de 60°C, sous un débit de 5,76 kg/heure, sous une pression de 1 bar.

Le raffinat sort en tête de l'extracteur sous un débit de 6,38 kg/heure et contient 98,9 % en poids d'ETBE et 1,1 % en poids d'éthanol.

L'extrait contenant 74,9 % en poids d'eau, 21,6 % en poids d'éthanol et 3,5 % en poids d'ETBE est envoyé sous un débit de 7,60 kg/heure dans la colonne de concentration au niveau du 6ème plateau.
La température de la colonne s'étale entre 99,5°C en fond et 73,5°C en tête.

Au fond de la colonne on recueille, sous un débit de 5,53 kg/heure, un produit contenant plus de 99,99 % d'eau et des traces d'éthanol.

Le distillat contenant 9,1 % en poids d'eau, 90,9 % en poids d'éthanol est condensé. Une fraction du condensat est renvoyée en tête de la colonne de concentration sous un débit de 4,68 kg/heure, à titre de reflux, le reste sous un débit de 1,81 kg/heure en tête de colonne à distiller CD1.

On introduit au départ 10 kg d'ETBE au décanteur.

La colonne CD1 est alimentée sur le 6ème plateau. La température de la colonne s'étale entre 78°C en fond et 64°C en tête.

En fond de la colonne CD1, on recueille, sous un débit de 1,66 kg/heure un produit contenant 99,1 % en poids d'éthanol, 0,6 % en poids d'ETBE et 0,3 % en poids d'eau.

Le distillat est réuni avec celui de la colonne de distillation CD2, leur mélange est condensé, puis envoyé au décanteur (débit liquide à l'entrée 15,9 kg/heure).

Du décanteur, on envoyé un reflux de phase supérieure (débit 15,5 kg/heure) vers la colonne CD1 et un reflux de phase inférieure (débit 0,4 kg/heure), vers la colonne CD2, sous la pression de 1 bar.

La température de la colonne CD2 s'étage entre 99,7°C en fond et 94,6°C en tête.

On recueille au fond de cette colonne, sous un débit de 0,16 kg/heure, un produit contenant plus de 99,99 % en poids d'eau et quelques ppm d'éthanol.

Ce produit, mélangé avec le produit de fond de la colonne de concentration, après un appoint d'eau éventuel, peut avantageusement constituer l'eau d'alimentation de l'extracteur.

## Revendications

1. Procédé de séparation d'éthyl tertiobutyl éther et d'éthanol, caractérisé en ce qu'il comprend :
(1) une étape d'extraction dans laquelle on introduit une charge consistant essentiellement en un mélange d'éthyl tertiobutyl éther et d'éthanol dans une zone d'extraction dans laquelle on introduit comme solvant d'extraction de l'eau et à partir de laquelle on recueille un raffinat consistant essentiellement en éthyl tertiobutyl éther saturé en eau et un extrait consistant en un mélange éthanol-eau contenant une faible proportion d'éthyl tertiobutyl éther;
(2) une étape de concentration dans laquelle l'extrait issu de l'étape (1) est envoyé dans une zone de concentration, à partir de laquelle on recueille en tête un distillat proche de la composition azéotropique éthanol-eau, en fond un résidu consistant essentiellement en eau, et on soutire une phase consistant essentiellement en éthyl tertiobutyl éther, qui est réunie avec le raffinat de l'étape d'extraction (1);
(3) une étape de distillation hétéroazéotropique, utilisant l'éthyl tertiobutyl éther comme agent azéotropant, dans laquelle on envoie le distillat de ladite étape de concentration dans une première zone de distillation à partir de laquelle on recueille un résidu constistant essentiellement en éthanol, le flux sortant en tête étant réuni au flux de tête d'une seconde zone de distillation, le flux résultant étant condensé et envoyé dans un décanteur où se séparent une phase supérieure riche en éthyl tertiobutyl éther envoyée en reflux en tête de ladite première zone de distillation et une phase inférieure riche en eau renvoyée en reflux en tête de ladite seconde zone de distillation, dont le résidu consiste essentiellement en eau, qui est envoyée, après réunion avec le résidu de ladite zone de concentration et éventuel appoint, en alimentation en tête de ladite zone d'extraction, de l'éthyl tertiobutyl éther étant introduit dans le décanteur lors de la mise en service du procédé.

2. Procédé selon la revendication 1, caractérisé en ce que la charge contient de 5 à 50% en poids d'éthanol.

3. Procédé selon la revendication 1, caractérisé en ce que la charge contient de 10 à 30% en poids d'éthanol.

4. Procédé selon l'une quelconque des revendications 1 à 3, caractérisé en ce que l'étape (1) on utilise une colonne d'extraction dans laquelle on opère à une pression de 1 à 2 bars, à une température de 50 à 70°C et avec un rapport d'extraction de 2 à 5 moles d'eau par mole de charge, en ce que dans l'étape (2) on utilise une colonne de concentration opérant à une pression de 1 à 2 bars, entre une température de fond de 100 à 120°C et une température de tête de 78 à 85°C, et en ce que, dans l'étape (3), on utilise dans la première zone de distillation une première colonne à distiller opérant à une pression de 1 à 2 bars , entre une température de fond de 78 à 85°C et une température de tête de 64 à 70°C, on utilise dans la deuxième zone de distillation une deuxième colonne à distiller opérant à une pression de 1 à 2 bars et entre une température de fond de 100 à 120°C et une température de tête de 94 à 110°C et le flux résultant de la réunion des flux de tête desdites colonnes à distiller est condensé par refroidissement à une température de 45 à 65°C, avant d'être amené dans le décanteur.

5. Procédé de séparation d'éthanol et d'eau, caractérisé en ce que le mélange éthanol-eau à séparer est traité par distillation hétéroazéotropique utilisant l'éthyl tertiobutyl éther comme agent azéotropant dans les conditions de l'étape (3) de la revendication 1.

6. Procédé selon la revendication 5, caractérisé en ce que, dans la première zone de distillation, on utilise une première colonne à distiller opérant à une pression de 1 à 2 bars et entre une température de fond de 78 à 85°C et une température de tête de 64 à 70°C; dans la deuxième zone de distillation, on utilise une deuxième colonne à distiller opérant à une pression de 1 à 2 bars et entre une température de fond de 100 à 120°C et une température de tête de 94 à 110°C; et le flux résultant de la réunion des flux de tête desdites colonnes à distiller est condensé par refroidissement à une température de 45 à 65°C avant d'être amené dans le décanteur.

7. Procédé de fabrication d'éthyl tertiobutyl éther par éthérification par de l'éthanol de l'isobutène contenu dans une coupe C₄ de vapocraquage, de craquage catalytique ou de déshydrogénation d'isobutane, dans lequel, dans une zone de réaction A, on met en contact dans des conditions réactionnelles de l'éthanol et ladite coupe C₄; le produit issu de ladite zone de réaction A, contenant principalement de l'éthyl tertiobutyl éther, de l'éthanol, des hydrocarbures en C₄ autres que l'isobutène et de l'isobutène non réagi, est envoyé dans une zone de distillation B où sont séparés en tête les hydrocarbures en C₄ incluant l'isobutène non réagi et en fond un mélange d'éthyl tertiobutyl éther et d'éthanol; on envoie ledit mélange dans une zone de séparation C; ledit procédé étant caractérisé en ce que, dans ladite zone de séparation C, on met en oeuvre un procédé selon l'une des revendications 1 à 4.

8. Procédé selon la revendication 7, caractérisé en ce que ladite zone de distillation B est remplacée par une zone de distillation réactive B', dans laquelle on introduit de l'éthanol pour éliminer l'isobutène non réagi.

9. Procédé selon la revendication 7, caractérisé en ce que l'éthanol purifié issu de la zone de séparation C est recyclé à l'entrée de la zone de réaction A.

10. Procédé selon la revendication 8, caractérisé en ce que l'éthanol purifié issu de la zone de séparation C est recyclé à l'entrée de la zone de réaction réactive B'.

## Claims

1. Process for separating ethyl tertiobutyl ether and ethanol, characterized in that it comprises:
(1) an extraction stage in which a charge, essentially comprising a mixture of ethyl tertiobutyl ether and ethanol, is introduced into an extraction zone into which water is introduced as extraction solvent and from which there is collected a raffinate, consisting essentially of ethyl tertiobutyl ether saturated in water, and an extract consisting in an ethanol/water mixture containing a small proportion of ethyl tertiobutyl ether;
(2) a concentration stage in which the extract from stage (1) is sent into a concentration zone from which a distillate close to the ethanol/water azeotropic composition is collected at the head, and a residue consisting essentially water at the bottom, and a phase consisting essentially of ethyl tertiobutyl ether is drawn off, and is combined with the raffinate from the extration stage (1); and
(3) a heteroazeotropic distillation stage using ethyl tertiobutyl ether as azeotroping agent, in which the distillate from the said concentration stage is sent into a first distillation zone from which a residue essentially comprising ethanol is collected, the flux emerging overhead being combined with the head flux from a second distillation zone, the resultant flux being condensed and sent into a decanter where separation takes place into an upper phase, rich in ethyl tertiobutyl ether, which is sent in reflux to the head of the said first distillation zone, and a lower phase, rich in water, which is returned in reflux to the head of the said second distillation zone, whose residue consists essentially of water, which is sent, after combining with the residue of the said concentration zone and possible make up, to feed the head of the said extraction zone, ethyl tertiobutyl ether being introduced into the decanter during the start-up of the process.

2. Process according to claim 1, characterized in that the charge contains from 5 to 50% by weight of ethanol.

3. Process according to claim 1, characterized in that the charge contains from 10 to 30% by weight of ethanol.

4. Process according to any one of claims 1 to 3, characterized in that in stage (1) an extraction column is used which is operated at a pressure of 2 bar, at a temperature of 50 to 70 degrees C with an extraction ratio of 2 to 5 moles of water per mole of charge, in that in stage (2) a concentration column is used which is operated at a pressure of 1 to 2 bar, between a bottom temperature of 100 to 120 degrees C and a head temperature of 78 to 85 degrees C, and in that, in stage (3), in the first distillation zone a first distillation column is used which is operated at a pressure of 1 to 2 bar, between a bottom temperature of 78 to 85 degrees C and a head temperature of 64 to 70 degrees C, in the second distillation is used which is operated at a pressure of 1 to 2 bar and between a bottom temperature of 100 to 120 degrees C and a head temperature of 94 to 110 degrees C and the flux resulting from the combining of the head fluxes of the said distillation columns is condensed by cooling to a temperature of 45 to 65 degrees C, before being fed into the decanter.

5. Process for separating ethanol and water, characterized in that the ethanol/water mixture to be separated is treated by heteroazeotropic distillation using ethyl tertiobutyl ether as azeotroping agent under the conditions of stage (3) of claim 1.

6. Process according to claim 5, characterized in that, in the first distillation zone, a first distillation is used which is operated at a pressure of 1 to 2 bar and between a bottom temperature of 78 to 85 degrees C and a head temperature of 64 to 70 degrees C; in the second distillation column is used, which is operated at a pressure of 1 to 2 bar and between a bottom temperature of 100 to 120 degrees C and a head temperature of 94 to 110 degrees C; and the flux resulting from the combining of the head fluxes of the said distillation columns is condensed by cooling to a temperature of 45 to 65 degrees C before being fed into the decanter.

7. Process for producing ethyl tertiobutyl ether through etherification by ethanol of the isobutene contained in a steam-cracking, catalytic cracking or isobutane dehydrogenation C₄ cut, in which, in a reaction zone A, is contacted with the said C₄ cut under reaction conditions; the product from the said reaction reaction zone A, containing principally ethyl tertiobutyl ether, ethanol, and C₄ hydrocarbons other than isobutene and non-reacted isobutene are separated overhead and a mixture of ethyl tertiobutyl ether and ethanol at the bottom; the said mixture is sent into a separation zone C; the said process being characterized in that, in the said separation zone C, a process according to one of claims 1 to 4 is implemented.

8. Process according to claim 7, characterized in that the said distillation zone B is replaced by a reactive distillation zone B' into which ethanol is introduced to eliminate the non-reacted isobutene.

9. Process acording to claim 7, characterized in that the purified ethanol form separation zone C is recycled to the entrance of reaction zone A.

10. Process acoording to claim 7, characterized in that the purified ethanol from separation zone C is recycled to the entrance of reactive distillation zone B'.

## Patentansprüche

1. Verfahren zum Trennen von Ethyl-tert.-Butylether und Ethanol, dadurch gekennzeichnet, daß es umfaßt:
(1) eine Extraktionsstufe, in der man eine Charge, die im wesentlichen aus einem Gemisch aus Ethyl-tert.-Butylether und Ethanol besteht, in eine Extraktionszone einführt, in die man als Extraktionslösungsmittel Wasser einführt, und aus der man ein Raffinat sammelt, das im wesentlichen aus mit Wasser gesättigtem, Ethyl-tert.-Butylether und einem Extrakt besteht, der aus einem Ethanol-Wasser-Gemisch besteht, welches einen geringen Anteil an Ethyl-tert.-Butylether enthält;
(2) eine Konzentrationsstufe, in der der aus der Stufe (1) stammende Extrakt in eine Konzentrationszone geschickt wird, aus der man am Kopf ein Destillat nahe der azeotropierenden Zusammensetzung Ethanol-Wasser sammelt, am Boden einen Rückstand, der im wesentlichen aus Wasser besteht und man eine Phase abzieht, die im wesentlichen aus Ethyl-tert.-Butylether besteht, das mit dem Raffinat der Extraktionsstufe (1) vereinigt wird;
(3) eine heteroazeotropierende Destillationsstufe, die den Ethyl-tert.-Butylether als azeotropierendes Mittel verwendet, in der man das Destillat aus dieser Konzentrationsstufe in eine erste Destillationszone schickt, aus der man einen Rückstand sammelt, der im wesentlichen aus Ethanol besteht, wobei der am Kopf austretende Strom vereinigt wird mit dem Kopfstrom einer zweiten Destillationsstufe, der resultierende Strom kondensiert und in eine Dekantiereinrichtung geschickt wird, wo sich eine obere ethyl-tert.-butyletherreiche Phase, die im Kopfrückstrom aus dieser ersten Destillationsstufe geschickt wird und eine untere wasserreiche Phase trennen, die im Rückstrom zum Kopf dieser zweiten Destillationszone geschickt wurde, deren Rückstand im wesentlichen aus Wasser besteht, das nach der Wiedervereinigung mit dem Rückstand dieser Konzentrationszone und gegebenenfalls einem Zusatz unter Speisung am Kopf dieser Extraktionszone von Ethyl-tert.-Butylether in die Dekantiervorrichtung bei der Durchführung dieses Verfahrens geschickt wird.

2. Verfahren nach Anspruch 1, dadurch gekennzeichnet, daß die Charge 5 bis 50 Gew.-% Ethanol enthält.

3. Verfahren nach Anspruch 1, dadurch gekennzeichnet, daß die Charge 10 bis 30 Gew.-% Ethanol enthält.

4. Verfahren nach einem der Ansprüche 1 bis 3, dadurch gekennzeichnet, daß man in der Stufe (1) eine Extraktionskolonne verwendet, in der man bei einem Druck von 1 bis 2 bar bei einer Temperatur von 50 bis 70°C und mit einem Extraktionsverhältnis von 2 bis 5 Mol Wasser pro Mol Charge arbeitet, daß man in der Stufe (2) eine Konzentrationskolonne verwendet, die bei einem Druck von 1 bis 2 bar zwischen einer Bodentemperatur von 100 bis 120°C und einer Kopftemperatur von 78 bis 85°C arbeitet und daß in der Stufe (3) man in der ersten Destillationszone eine erste Destillationskolonne verwendet, die bei einem Druck von 1 bis 2 bar zwischen einer Bodentemperatur von 78 bis 85°C und einer Kopftemperatur von 64 bis 70°C arbeitet, man in der zweiten Destillationszone eine Destillierkolonne verwendet, die bei einem Druck von 1 bis 2 bar und zwischen einer Bodentemperatur von 100 bis 120°C und einer Kopftemperatur von 94 bis 110°C arbeitet und der resultierende Fluß aus der Vereinigung der Kopfströme dieser Destillierkolonnen durch Kühlen auf eine Temperatur von 45 bis 65°C vor Rückführung in die Dekantiervorrichtung kondensiert wird.

5. Verfahren zum Trennen von Ethanol und Wasser, dadurch gekennzeichnet, daß das zu trennende Ethanol-Wasser-Gemisch behandelt wird durch heteroazeotropierende Destillation unter Verwendung von Ethyl-tert.-Butylether als azeotropierendes Mittel unter den Bedingungen der Stufe (3) von Anspruch 1.

6. Verfahren nach Anspruch 5, dadurch gekennzeichnet, daß in der ersten Destillationszone man eine erste Destillierkolonne verwendet, die bei einem Druck von 1 bis 2 bar und zwischen einer Bodentemperatur von 78 bis 85°C und einer Kopftemperatur von 64 bis 70°C arbeitet; man in der zweiten Destillationszone eine zweite Destillierkolonne verwendet, die bei einem Druck von 1 bis 2 bar und zwischen einer Bodentemperatur von 100 bis 120°C und einer Kopftemperatur von 94 bis 110°C arbeitet und der aus der Vereinigung der Kopfströme dieser Destillierkolonnen resultierende Fluß kondensiert wird durch Kühlen auf eine Temperatur von 45 bis 65°C vor der Rückführung in die Dekantiervorrichtung.

7. Verfahren zum Herstellen von Ethyl-tert.-Butylether durch Veretherung mit Isobutenethanol, das in einer C₄-Fraktion der Dampfkrackung, der katalytischen Krackung oder der Isobutandehydrierung enthalten ist, bei dem in einer Reaktionszone A man unter Reaktionsbedingungen Ethanol und diese C₄-Fraktion kontaktiert; das aus dieser Reaktionszone A stammende Produkt, das hauptsächlich Ethyl-tert.-Butylether, Ethanol, C₄-Kohlenwasserstoffe außer Isobuten und nicht umgesetztes Isobuten enthält, in eine Destillationszone B gechickt wird, wo am Kopf die nicht umgesetztes Isobuten einschließenden C₄-Kohlenwasserstoffe und am Boden ein Gemisch von Ethyl-tert.-Butylether und Ethanol abgetrennt werden; man dieses Gemisch in eine Trennzone C schickt; wobei das Verfahren sich dadurch auszeichnet, daß in dieser Trennzone C man ein Verfahren gemäß einem der Ansprüche 1 bis 4 verwirklicht.

8. Verfahren nach Anspruch 7, dadurch gekennzeichnet, daß diese Destillationszone B ersetzt wird durch eine Reaktivdestillationszone B', in der man Ethanol zur Eliminierung von nicht umgesetztem Isobuten einführt.

9. Verfahren nach Anpruch 7, dadurch gekennzeichnet, daß das aus der Trennzone C stammende gereinigte Ethanol an den Eingang der Reaktionszone A recycliert wird.

10. Verfahren nach Anspruch 8, dadurch gekennzeichnet, daß das aus der Trennzone C stammende gereinigte Ethanol an den Eingang der reaktiven Reaktionszone B' recycliert wird.
